Europäisches Patentamt

**European Patent Office**

**Office européen des brevets**

(19)

(11) Veröffentlichungsnummer: **0 065 257**
A2

(12)

# EUROPÄISCHE PATENTANMELDUNG

(21) Anmeldenummer: **82104060.7**

(22) Anmeldetag: **11.05.82**

(51) Int. Cl.³: **C 12 N 15/00, C 12 P 35/00**

(30) Priorität: **14.05.81 DE 3119196**

(43) Veröffentlichungstag der Anmeldung: **24.11.82**
**Patentblatt 82/47**

(84) Benannte Vertragsstaaten: **CH DE FR GB IT LI**

(71) Anmelder: **HOECHST AKTIENGESELLSCHAFT,
Postfach 80 03 20, D-6230 Frankfurt/Main 80 (DE)**

(72) Erfinder: **Minuth, Walter, Brüder-Grimm-Strasse 32,
D-6000 Frankfurt am Main 60 (DE)**
Erfinder: **Esser, Karl, Prof. Dr., Am Spik 23a,
D-4630 Bochum (DE)**
Erfinder: **Mracek, Miroslav, Ulmenstrasse 45,
D-6000 Frankfurt am Main (DE)**
Erfinder: **Nesemann, Georg, Dr., Bornstrasse 73,
D-6238 Hofheim am Taunus (DE)**

(54) **Verfahren zur Gewinnung neuer, zur Antibiotika-Herstellung geeigneter Pilzstämme.**

(57) Verfahren zur Gewinnung neuer, zur Antibiotikaherstellung geeigneter Pilzstämme durch Protoplasten- und Kernverschmelzung, bei dem man Protoplasten von Pilzstämmen unterschiedlicher Arten oder unterschiedlicher Gattungen in einer 10 bis 40 Gew.-% Polyäthylenglykol enthaltenen, isotonischen Lösung bei einem pH zwischen 6 und 8,5 mischt und die dabei entstehenden Hybridstämme in an sich bekannter Weise herausselektioniert.

EP 0 065 257 A2

HOECHST AKTIENGESELLSCHAFT   HOE 81/F 112   Dr.Kl /cr

0065257

Verfahren zur Gewinnung neuer, zur Antibiotika-Herstellung geeigneter Pilzstämme

Gegenstand der Erfindung ist ein Verfahren zur Gewinnung neuer Pilzstämme, mit denen Antibiotika hergestellt werden können, durch Protoplasten- und Kernverschmelzung.

Es ist aus der deutschen Offenlegungsschrift 29 16 736 bekannt, daß man verschiedene Stämme von Acremonium chrysogenum durch Protoplasten- und Kernverschmelzung kreuzen kann. Dadurch können neue Stämme von A.chrysogenum erhalten werden, die besonders vorteilhafte Eigenschaften der beiden Mutterstämme auf sich vereinigen und Cephalosporin C in guten Ausbeuten produzieren können.

Bei diesem bekannten Verfahren sind jedoch lediglich verschiedene Stämme der gleichen Art, nämlich von A.chrysogenum gekreuzt worden. Die Methode der Protoplasten- und Kernverschmelzung ist bisher noch nicht dafür eingesetzt worden, Pilzstämme unterschiedlicher Arten oder sogar unterschiedlicher Gattungen zu kreuzen und so neue Pilzstämme zu erhalten, die zur Antibiotika-Herstellung vorteilhaft eingesetzt werden können.

Es stellte sich deshalb die Aufgabe, aus bekannten, Antibiotika bildenden Pilzstämmen durch Protoplasten- und Kernfusion mit Pilzstämmen anderer Arten oder sogar anderer Gattungen neue Pilzstämme zu gewinnen, aus denen verbesserte Hochleistungsstämme herausselektioniert werden können. Von ganz besonderem Interesse sind dabei Kreuzungsprodukte von ß-Lactam-Antibiotika erzeugenden Pilzstämmen. Hierbei kann man es sich zu Nutze machen, daß eine recht große Anzahl von Pilzstämmen bekannt ist, die über die Fähigkeit zur Bildung von ß-Lactam-Antibiotika verfügen, aber nur wenige davon kommerziell eingesetzt werden, wie A.chrysogenum oder Penicillium chrysogenum. Gerade solche bisher noch nicht für die technische Herstellung von ß-Lactam-Antibiotika verwendete Stämme, z.B. andere Acremonium-Arten, aber auch Vertreter anderer Gattungen wie Paecilomyces, Emericellopsis oder Penicillium-Arten können als aussichtsreiche Kombinationspartner für z.B. A.chrysogenum angesehen werden, falls bei derartigen Fusionen von Pilzstämmen unterschiedlicher Arten oder sogar unterschiedlicher Gattungen überhaupt noch lebens- und wachstumsfähige Hybride entstehen.

Es wurde nun gefunden, daß sich zur Antibiotika-Herstellung geeignete Pilzstämme durch Protoplasten- und Kernverschmelzung gewinnen lassen, wenn man Pilzstämme unterschiedlicher Arten oder unterschiedlicher Gattungen in einer isotonischen, 10 bis 40 Gew.-% Polyäthylenglykol enthaltenden Lösung bei einem pH zwischen 6 und 8,5 mischt und die dabei entstehenden Hybrid-Stämme in an sich bekannter Weise herausselektioniert.

Als Polyäthylenglykol eignet sich grundsätzlich jedes, das die erforderliche Löslichkeit in der wäßrigen isotonischen Lösung aufweist. Im allgemeinen nimmt man ein Produkt mit einem mittleren Molgewicht von etwa 1000 bis etwa 10 000.

Die Protoplasten der beiden zur Verschmelzung vorgesehenen Mutterstämme werden gewonnen, in dem man die Myzelien beider Stämme getrennt voneinander in Schüttelkolben in einer geeigneten Nährlösung nach an sich bekannter Weise kultiviert. Gegen Ende der Wachstumsphase haben sich große Mengen fädiger Myzelien gebildet, die aus dem Kulturmedium abfiltriert und gründlich gewaschen werden.

Zur Erhöhung der Protoplasten-Ausbeute werden dann die Myzelien bis zu 3 Stunden, vorzugsweise 1/2 bis 2 Stunden lang, bei 20 bis 40°C und in einem pH-Bereich zwischen 6 und 8,5 in einer isotonischen Lösung, die 0,3 bis 3 Gew.-%, vorzugsweise 0,5 Gew.-%, 2-Mercaptoäthanol enthält, suspendiert. Durch diese Behandlung steigt die Protoplastenausbeute auf den 2- bis 3-fachen Wert gegenüber einem unbehandelten Ansatz.

Die so vorbehandelten Myzelien werden nun der Einwirkung von die Zellwände abbauenden Enzymen unterworfen, um die Protoplasten isolieren zu können. Geeignete Enzyme hierfür, z.B. der Trichoderma harzianum-Enzymkomplex, das Cytophaga Enzym $L_1$, Sulfatase und Zymolase können käuflich erworben werden und werden einzeln oder in Mischung bei neutralem pH und einer Temperatur zwischen 24 und 35°C in einem isotonischen Medium eingesetzt. Zweckmäßig wird die Myzel-Suspension dabei geschüttelt. Der Abbau der Zellwände erfordert für unterschiedliche Pilzkulturen auch unterschiedliche Zeiten, ist aber nach 30 bis 360 Min. in der Regel im wesentlichen abgeschlossen.

Im allgemeinen werden bei diesem Verfahren aus in 20 ml Medium suspendierten 1 g Myzelium etwa $10^5$ bis $10^6$ Proto-plasten/ml gewonnen. Diese können nach dem Waschen - z.B. in einer isotonischen, 10 bis 30 Gew.-% Glycerin ent-haltenden Lösung - und Abtrennen restlicher Myzel-Stücke bei -20°C etwa 10 Tage regenerationsfähig gehalten werden.

Zur Verschmelzung der Protoplasten werden nun etwa gleiche Mengen beider Protoplasten-Sorten miteinander vermischt und etwa 5 Min. zentrifugiert. Das Sediment wird dann in einer 10 bis 40 Gew.-%, vorzugsweise 20 bis 30 Gew.-% Polyäthylenglykol enthaltenden, neutralen und isotonischen Lösung suspendiert. Der Zusatz von Polyäthylenglycol erfolgt hierbei, um die sonst sehr geringe Verschmelzungsrate der Protoplasten zu steigern. Bevorzugt wird hierfür ein Polyäthylenglykol mit einem Molekulargewicht von 2000 bis 6000. Eine weitere Verbesserung der Protoplasten-Verschmelzung wird schließlich durch den Zusatz von Calciumionen erreicht. Deshalb ist die Zugabe einer 0,1 bis 0,5 molaren Calciumchlorid-Lösung von Vorteil.

Besonders gute Resultate werden erhalten, wenn man eine oder beide Protoplastensuspensionen vor der Verschmelzung einige Minuten unter ständigem Rühren mit UV-Licht bestrahlt.

Die so vorbehandelte Protoplasten-Mischung wird dann bei 24 bis 35°C etwa 5 bis 20 Minuten, vorzugsweise etwa 10 Minuten inkubiert und dann das Polyäthylenglykol durch Zentrifugieren abgetrennt. Die nunmehr fusionierten Protoplasten werden dann in einer isotonischen, neutralen Lösung resuspendiert und zur Stimulierung der Kernfusionsfrequenz erneut für 5 bis 20 Sekunden mit UV-Licht in einer Petrischale bestrahlt.

Die nach diesem Verfahren entstandenen Hybride können nun in an sich bekannter Weise durch Selektion gefunden und dann kultiviert werden. Hierbei ist es von großem Nutzen, wenn genetisch markierte Mutterstämme zur Verfügung stehen, die sich durch besondere Auxotrophien, Farben oder Arzneimittelresistenzen auszeichnen. Weist z.B. einer der Mutterstämme eine Auxotrophie für bestimmte Aminosäuren wie Histidin, Methionin oder Arginin auf, dann wird er auf einem diese Aminosäuren nicht enthaltenden Minimalmedium nicht wachsen, während der durch Kernverschmelzung entstandene Hybrid wegen seiner andersartigen genetischen Konstitution von diesen Aminosäuren nicht abhängig zu sein braucht und deshalb unbehindert wachsen kann.

Ebenso können Arzneimittelresistenzen die Selektion erleichtern. Verfügt nur einer der beiden Mutterstämme über eine Resistenz gegenüber einem bestimmten Arzneimittel und wird diese Resistenz auf den Hybrid-Stamm übertragen, dann kann der andere Mutterstamm, der auf einem ein derartiges Arzneimittel enthaltenden Medium nicht wächst, abgetrennt werden.

0065257

Der durch derartige Methoden in reiner Form hergestellte
Hybridstamm kann dann auf einem geeigneten Nährmedium
kultiviert und das von ihm gebildete Antibiotikum nach
bekannten Verfahren isoliert werden.

Von allen Kreuzungsprodukten wurde nach der Agarstück-
Methode die ß-Lactam-Produktion bestimmt und mit der der
Mutterstämme verglichen. Dabei zeigte sich in einigen
Fällen eine erhöhte ß-Lactam-Produktion der erfindungsgemäß
hergestellten Hybride. Damit ist die grundsätzliche
Eignung dieser genetischen Methode für die Stammverbesserung
nachgewiesen. Ebenso konnte bei einigen Hybriden eine
deutliche Verbesserung der Sporulation gefunden werden.

0065257

**Beispiel 1**

Eine aus dem Stamm A.chrysogenum (ATCC 14 553) abgeleitete Linie mit den genetischen Markierungen his-, met- und BL$^+$ und eine Acremonium species (ATCC 18 739) mit den genetischen Markierungen arg$^-$, nys$^{res}$, gri$^{res}$, sp$^+$ und chr$^+$ wurde für die im folgenden beschriebene Kreuzung zweier unterschiedlicher Acremonium-Arten verwendet. Die Abkürzungen his$^-$, met$^-$ und arg$^-$ bedeuten Auxotrophien für Histidin, Methionin und Arginin, nys$^{res}$ die Resistenz gegen Nystatin, gri$^{res}$ die Resistenz gegen Griseofulvin, BL$^+$ die Fähigkeit der ß-Lactam Antibiotika-Bildung, sp$^+$ die Sporulationsfähigkeit und chr$^+$ die Produktion des gelben Pigments Chrisogenin.

Die beiden Stämme wurden im folgenden flüssigen Medium getrennt voneinander vorgezüchtet:

| | |
|---|---|
| Bactoextract Difco | 0,3 % |
| Glukose | 0,5 % |
| Natriumchlorid | 0,5 % |
| Aqua dest | |
| pH 6,8 | |

Von diesem Medium wurden jeweils 50 ml in zwei 300 ml Erlenmeyerkolben abgefüllt und sterilisiert. In den einen Erlenmeyerkolben wurden ca. 50 cm$^2$ eines 10-12 Tage alten Myzeliums übertragen, das aus der aus ATCC 14 553 gewonnenen Linie in einer Rouxflaschenkultur gebildet worden war. Dieses Myzel wurde nun bis zum Auskeimen der Arthrosporen etwa 50 bis 60 Stunden lang auf einer Rotationsschüttelmaschine kultiviert.

In den zweiten Erlenmeyerkolben wurde ca. 1 cm$^2$ eines 10 bis 12 Tage alten Myzeliums der Acremonium species (ATCC 18 739) aus einer Stockkultur übertragen und 30 bis 40 Stunden kultiviert. Nach der Beendigung der Vorkultivierung wurden die beiden Myzelien einzeln durch Filtration oder Zentrifugieren von der Kulturlösung ge-

0065257

trennt, zweimal mit destilliertem Wasser gewaschen, in einer isotonischen, neutralen, 0,5 % 2-Mercaptoäthanol enthaltenden Lösung suspendiert und bei 30°C 30 Min. stationär inkubiert. Danach werden die Myzelien mit einer isotonischen, neutralen Lösung gewaschen.

Die eigentliche Protoplastenbildung erfolgt bei der aus A.chrysogenum (ATCC 14 553) gewonnenen Linie durch die Einwirkung von 12 mg/ml des Cytophaga-Enzyms $L_1$ und von 2 mg/ml Zymolase in einer isotonischen, neutralen Lösung in einem 200 ml Erlenmeyerkolben bei leichtem Schütteln auf einer Schüttelmaschine und einer Temperatur von 30°C im Verlaufe einer etwa dreistündigen Inkubation.

Die Protoplasten von Acremonium species (ATCC 18 739) werden in ähnlicher Weise gewonnen, nur werden hier 4 mg/ml des Trichoderma harzianum-Enzymkomplexes und 1,5 mg/ml Sulfatase eingesetzt.

Die Protoplasten wurden dann mit Hilfe einer Filtration (Glaswattefilter) von den restlichen Myzelstücken getrennt und zweimal durch eine 5 minütige Zentrifugation in isotonischer Lösung gewaschen. Der Protoplastentiter soll bei ca. $10^6$ Protoplasten/ml liegen. Kurz vor der Protoplastenfusion werden 4 ml der Protoplastensuspension des Stammes A.species (ATCC 18 739) in einer Petrischale unter ständigem Rühren 6 Minuten mit UV-Licht einer Wellenlänge von 254 nm bestrahlt.

2 ml von jeder der beiden Protoplastensuspensionen wurden zusammengeführt, durchgemischt und 5 Minuten zentrifugiert. Das Sediment wurde in 0,2 ml einer 0,5 molaren Calciumchloridlösung suspendiert, auf 2 ml Polyäthylenglykollösung (bestehend aus 30 Gew.-% Polyäthylenglykol mit einem Molekulargewicht von 6000 und 100 mM Calciumchlorid, pH 7,5) aufgetragen und 10 Minuten bei 30°C inkubiert. Nach Zugabe von 5 ml einer wässrigen isotonischen Lösung von pH 6,7 wird durch 5 minütige Zentrifugation das Polyäthylenglykol abgetrennt. Die fusionierten Protoplasten werden in einer isotonischen, neutralen Lösung suspendiert und zur Stimulierung der Kernfusionsfrequenz 10 Sekunden mit UV-Licht einer Wellenlänge von 254 nm in einer Petrischale bestrahlt. Die Verschmelzungsprodukte wurden auf einem kompletten und auf einem minimalen Selektionsmedium kultiviert.

Das Minimalmedium hatte folgende Zusammensetzung:

| | |
|---|---|
| Glukose | 40 g |
| NaNO$_3$ | 3 g |
| KH$_2$PO$_4$ · 7H$_2$O | 0,5 g |
| FeSO$_4$ · 7H$_2$O | 0,01 g |
| NaCl | 35,0 g (0,6 M) |
| Agar (Serva ) | 20 g |
| dest. H$_2$O | ad 1000 ml |

Dieses Medium wurde 20 Minuten bei 121°C sterilisiert. Der pH-Wert stellte sich auf 7,1 - 7,2 ein.

Das Komplettmedium hatte folgende Zusammensetzung:

| | |
|---|---|
| Saccharose | 3,0 g |
| Dextrin (weiß) | 15,0 g |
| NaCl | 35,0 g (0,6 M) |
| K$_2$HPO$_4$ | 0,5 g |
| MgSO$_4$ · 7H$_2$O | 0,5 g |
| FeSO$_4$ · 7H$_2$O | 0,01 g |
| Trypticase-Soy-Broth (Difco) | 5,0 g |
| Fleischextract (Difco) | 1,0 g |
| Hefeextrakt (Difco) | 2,0 g |
| Agar (Serva) | 20 g |
| Dest H$_2$O | ad 1000 ml |

Dieses Medium wurde 20 Minuten bei 121°C sterilisiert. Der pH-Wert stelle sich hier auf 7,0 - 7,2 ein.

- 9 -

0065257

Nach 3 Passagen auf dem Minimalmedium und 2 Passagen
auf dem Komplettmedium wurden eine Reihe von Kulturen
erhalten, von denen einige charakterisiert wurden.

Unter den Fusionsprodukten wurden die Stämme IS-1
und IS-2 als neue Rekombinanten gefunden. Die nachfolgende Tabelle zeigt die charakteristischen Eigenschaften dieser neuen Stämme im Vergleich zu ihren
Mutterstämmen:

| Stamm | Sporu-lation * | chr | Wachs-tum ** | Nys | Gri | his | met | arg |
|---|---|---|---|---|---|---|---|---|
| Linie aus A-chrysogenum (ATCC 14 553) | $10^3$ | $\pm$ | 6,2 | sens | sens | - | - | + |
| A. species (ATCC 18 739) | $10^8$ | +++ | 14,5 | res | res | + | + | - |
| IS-1 | $10^2$ | $\pm$ | 14,2 | res | res | + | + | + |
| IS-2 | $10^7$ | - | 14,6 | res | sens | + | + | + |

* Sporenbildung, d.h. durchschnittliche Zahl der Sporen/
ml nach der 10 ml Abschwemmung von 10 $cm^2$ 14 Tagealten Myzelium auf dem Komplettmedium gewachsen

** Wachstum in Submerskultur, g Trockengewicht/l nach
4 Tagen Kultivation, Komplettmedium, Rotationsschüttelmaschine 250 Upm, 25°C.

- keine Bildung von Chrysogenin
$\pm$ Spuren von Chrysogenin
+++ ausgeprägte Bildung von Chrosogenin

**Beispiel 2**

Genetisch markierte Stämme der Art Emericellopsis glabra
(CBS 11 940) mit den Merkmalen phe⁻ und try⁻ und
Emericellopsis salmosynnemata (CBS 18 256) mit den
Merkmalen met⁻ und nia⁻ wurden analog der im Beispiel 1
beschriebenen Methode rekombiniert. Die Abkürzungen phe⁻,
try⁻, met⁻ und nia⁻ bedeuten hierbei Auxotrophien für
Phenylalanin, Tryptophan, Methionin und Nikotinsäureamid.
Für die Protoplastenherstellung wurde die Enzymkombination
von 6 mg/ml Trichoderma harzianum Enzymkomplex und 2 mg/ml
Sulfatase verwendet.

In der nachfolgenden Tabelle sind die gefundenen
Rekombinanten durch die beobachteten Auxotrophien und
ihre Morphologie gekennzeichnet.

| Rekombinanten | Zahl der Isolate | Morphologie |
|---|---|---|
| phe⁻, try⁻, nia⁻ | 1 | Emericellopsis glabra |
| phe⁻, nia⁻ | 2 | " " |
| try⁻, nia⁻ | 1 | " " |
| phe⁻ | 4 | " " |
| try⁻ | 5 | " salmosynnemata |
| met⁻, phe⁻ | 1 | " " |
| met⁻ | 6 | " " |
| nia⁻ | 10 | " " |

Hiermit konnte gezeigt werden, daß durch Protoplastenverschmelzung eine Kreuzung zwischen verschiedenen
Emericellopsis-Arten möglich ist.

**Beispiel 3**

Zum Nachweis der Kreuzungsmöglichkeit zwischen Pilzstämmen
unterschiedlicher Gattungen wurde eine genetisch markierte
Linie von Acremonium chrysogenum (ATCC 14 553) mit den
genetischen Merkmalen met⁻, pyr⁻ und BL⁺ und
Emericellopsis glabra (CBS 11 940) mit den genetischen
Merkmalen phe⁻, try⁻, ben^res rekombiniert. Hierbei bedeuten met⁻, pyr⁻, phe⁻, und try⁻ Auxotrophien für Methionin, Pyridoxin, Phenylalanin und Tryptophan.
$BL^+$ bedeutet die Fähigkeit zur Bildung von ß-Lactam-
Antibiotika und ben$^{res}$ bedeutet eine Resistenz gegen
Benomyl.

Für die Protoplastenisolierung wurde die Enzymkombination
aus 6 mg/ml Trichoderma harzianum Enzym und 3 mg/ml
Sulfatase verwendet, im übrigen aber wie in den beiden
vorhergehenden Beispielen vorgegangen.

Die Charakteristika der intergenerischen Rekombinanten
zeigt die folgende Tabelle:

| Rekombinanten | Zahl der Isolate | Morphologie |
|---|---|---|
| met$^-$, phe$^-$, ben$^{res}$ | 4 | Emericellopsis glabra |
| met$^-$, try$^-$, ben$^{res}$ | 2 | " " |
| met$^-$, try$^-$ | 1 | " " |
| met$^-$, ben$^{res}$ | 2 | " " |
| phe$^-$, ben$^{res}$ | 6 | " " |
| try$^-$, ben$^{res}$ | 4 | " " |
| phe$^-$, try$^-$ | 6 | " " |

Diese Ergebnisse zeigen, daß Kreuzungen zwischen Pilzstämmen unterschiedlicher Gattungen wie Acremonium und
Emericellopsis durch Protoplastenverschmelzung möglich
sind. Eine Übertragung der ß-Lactam-Bildung wurde bisher
nicht nachgewiesen. Dennoch ist bemerkenswert, daß dieses
Kreuzungsexperiment überhaupt glückte.

Beispiel 4

Zur intergenerischen Kreuzung wurde eine Linie von Acremonium chrysogenum (ATCC 14 553) und dem genetischen Merkmal met⁻ und eine von Penicillium chrysogenum (ATCC 9480) abgeleitete Linie mit den genetischen Merkmalen ben$^{res}$ und cb$^{res}$ eingesetzt. Hierbei bedeuten met⁻ eine Auxotrophie für Methionin und ben$^{res}$ eine Resistenz gegen Benomyl und cb$^{res}$ eine Resistenz gegen Carbendazim . Mit Hilfe der in den vorhergehenden Beispielen beschriebenen Protoplastenfusion wurde hier eine prototrophe Rekombinante gewonnen, die die genetischen Merkmale ben$^{res}$ und cb$^{res}$ trug, in ihrer Morphologie aber zwischen Acremonium chrysogenum und Penicillium chrysogenum lag.

PATENTANSPRÜCHE:

1. Verfahren zur Gewinnung neuer, zur Antibiotikaherstellung geeigneter Pilzstämme durch Protoplasten- und Kernverschmelzung, dadurch gekennzeichnet, daß man Protoplasten von Pilzstämmen unterschiedlicher Arten oder unterschiedlicher Gattungen in einer 10 bis 40 Gew.-% Polyäthylenglykol enthaltenden, isotonischen Lösung bei einem pH zwischen 6 und 8,5 mischt und die dabei entstehenden Hybridstämme in an sich bekannter Weise herausselektioniert.

2. Verfahren nach Anspruch 1, dadurch gekennzeichnet, daß man die Protoplasten zur Erhöhung der Protoplastenfusionsrate und zur Erhöhung der Kernfusionsrate mit einer subletalen Dosis UV-Licht bestrahlt.

3. Verfahren nach Anspruch 1 und 2, dadurch gekennzeichnet, daß man die Protoplastenfusion in Gegenwart von Calcium-Ionen durchführt.

4. Verfahren nach Anspruch 1 bis 3, dadurch gekennzeichnet, daß man Protoplasten verschiedener Pilzspecies fusioniert.

5. Verfahren nach Anspruch 1 bis 3, dadurch gekennzeichnet, daß man Protoplasten verschiedener Pilzgenera fusioniert.

6. Verfahren nach Anspruch 4 oder 5, dadurch gekennzeichnet, daß man genetisch markierte Pilzstämme fusioniert.

7. Verfahren nach Ansprüchen 1 bis 6, dadurch gekennzeichnet, daß man ß-Lactam-Antibiotika bildende Pilzstämme miteinander fusioniert.

HOE 81/F 112

8. Verfahren nach Anspruch 1 bis 7, dadurch gekennzeichnet, daß das Polyäthylenglykol ein mittleres Molgewicht von 1 000 bis 10 000 hat.

9. Verfahren nach Anspruch 1 bis 8, dadurch gekennzeichnet, daß das Polyäthylenglykol ein mittleres Molgewicht von 2 000 bis 6 000 hat.